# EUROPEAN PATENT APPLICATION

(11) **EP 1 974 730 A1**
(43) Date of publication of application: **01.10.2008**
(21) Application number: 08160014.0
(22) Date of filing: 03.11.2004
(51) Int. Cl.: A61K 31/437, A61P 1/04

(54) **Imidazo[1,2-a]pyridine derivatives for use in the treatment of sleep disturbance due to silent gastro-esophageal reflux**

(30) Priority: 03.11.2003 US 517125 P
(62) Divisional of application: 04800252.1
(71) Applicant: AstraZeneca AB, 151 85 Södertälje (SE)
(72) Inventor: Fernström, Paula, 431 83, Mölndal (SE); Hasselgren, Göran, 431 83, Mölndal (SE)

(57) **Abstract**

The present invention relates to a new method of treatment of sleep disturbance due to silent gastro-esophageal reflux. In particular, the present invention relates to the use of certain imidazo[1,2-a]pyridines derivatives in said treatment.

## Description

### Field of the Invention

The present invention relates to a new method of treatment of sleep disturbance due to silent gastro-esophageal reflux. In particular, the present invention relates to the use of certain imidazo[1,2-a]pyridines derivatives in said treatment.

### Background and Detailed Disclosure of the Invention

It is well established that recurrent symptomatic reflux episodes negatively influences sleep quality. Patients that have both upright and supine reflux (day and night time) have more extensive esophageal mucosal damage than the upright (daytime) refluxers. Reduced saliva production, decreased number of swallows and delayed esophageal clearance time during sleep contribute to the more extensive esophageal mucosal damage. Sleep is affected in about 50-60% of patients with gastroesophageal reflux disease (GERD). These patients may be functionally impaired during the day as a consequence of their symptoms/sleep disturbances.

It is known that infusion of acid even in non-GERD subjects causes arousals (Orr et al, Gastroenterology, 86, 814-819, 1984.) Patients could thus present to the physician with sleep disturbances but without symptoms of GERD. About 5% of the US population have sleep disturbances and it is estimated that 50% of these may have underlying acid reflux that is disrupting their sleep. The sleep problems may erroneously lead to prescription of hypnotics. Since hypnotics suppress arousals a hypnotic could actually increase acid contact time since it has been shown that the acid clearance is progressively and extensively prolonged during deeper sleep levels (Orr, 1984). This may ultimately increase the risk of developing esophagitis (Johnson, DeMeester, American Journal of Digestive Diseases, 23(6), 498-509, 1978), ulceration of the esophagus, Barrett's esophagus and similar conditions.

The target patient population also includes those who have fragmented sleep with frequent arousals. Sleep causes partial amnesia, which implies that even if patients cannot recall having had a reflux episode, the reflux episode may still be the reason for disrupting/fragmenting the sleep.

A similar mechanism is seen in patients with reflux laryngitis or acid asthma among which, less than 50% actually experience heartburn.

The side effects with hypnotics are well established (i.e., addiction, decreased quality of life and productivity) and other treatments to address sleep disturbance are needed.

The present invention relates to sleep disturbance due to silent gastro-esophageal reflux , and this should be clearly differentiated from the nocturnal GERD/heartburn related sleep disturbance, since these patients are excluded.

The present invention thus relates to the treatment of patients with sleep disturbance due to silent gastro-esophageal reflux by administering a therapeutically effective amount of certain imidazo[1,2-a]pyridines derivatives.

In order words the present invention relates to the treatment of sleep disturbance due to silent gastro-esophageal reflux, i.e. the patient does not experience heartburn symptoms or other typical or traditional reflux symptoms, e.g. regurgitation. The patient may awaken, or get a change in sleep level (arousals) in response to the reflux event.

We have surprisingly found that reflux of acidic contents into the esophagus causes arousals/awakening even if the reflux episode is not associated with heartburn, regurgitation or acid taste. This results in sleep disturbance with reduced sleep quality with reduced quality of life and productivity.

Consequently the present invention offers a unique feature by i) improving sleep ii) reduce the risk of developing esophagitis iii) prevent development of Barretts' esophagus / adeno carcinoma, and iv) ultimately reduce the use of hypnotics in this group of patients.

The present invention is the first to disclose the relation between endogenous acid secretion and sleep disturbance and / or arousels and to link in time link the arousels to the EEG, EOG, EMG, and/or EKG of the patient.

### The active ingredient

A number of novel pharmaceutical agents are currently undergoing clinical evaluation for the treatment of gastro-oesophageal reflux disease. These include transient lower oesophageal sphincter relaxation-reducing agents, serotonergic agents/prokinetics, potassium-competitive acid blockers, mucosal protectants, histamine H3 agonists and anti-gastrin agents (N Vakil, Alimentary Pharmacology & Therapeutics Volume 19 Issue 10 Page 1041 - May 2004). Surprisingly, it has now been found that potassium-competitive acid blockers (P-CAB), whose original field of use is the treatment of gastric and intestinal disorders, are particularly suitable for the treatment of sleep disturbance due to silent gastro-esophageal reflux.

The present invention thus relates in a first aspect to the use potassium-competitive acid blockers in the treatment of sleep disturbance due to silent gastro-esophageal reflux.

P-CABs are designated as those substances which inhibit the enzyme responsible for gastric acid secretion. The term "P-CAB or P-CABs" according to the present invention comprises not only the active compounds as such, but also their pharmacologically acceptable salts and solvates (in particular hydrates) etc. P-CABs are disclosed and claimed e.g. in the following patent applications; WO 9837080, WO 9928322, WO 9955706, WO 9955705, WO 0010999, WO 0011000, WO 02060440, WO 02060441, WO 02060442, WO 03018582 (all AstraZeneca).

One aspect of the present invention is thus to administer to a subject suffering from sleep disturbance due to silent gastro-esophageal reflux a therapeutically effective amount of certain certain imidazo[1,2-a]pyridines derivatives. Examples of such imidazo[1,2-a]pyridines derivatives are those of Formula I 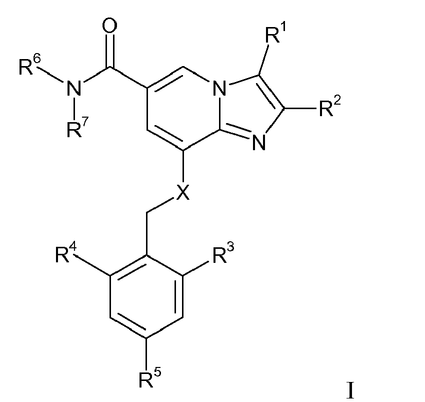 or a pharmaceutically acceptable salt thereof, wherein
R¹ is
(a) H,
(b) CH₃, or
(c) CH₂OH;
R² is
(a) CH₃
(b) CH₂CH₃
R³ is
(a) H
(b) C₁-C₆ alkyl,
(c) hydroxylated C₁-C₆ alkyl
(d) halogen
R⁴ is
(a) H,
(b) C₁-C₆ alkyl,
(c) hydroxylated C₁-C₆ alkyl, or
(d) halogen;
R⁵ is
(a) H, or
(b) halogen;
R⁶, R⁷ are the same or different
(a) H,
(b) C₁-C₆ alkyl;
(c) hydroxylated C₁-C₆ alkyl
(d) C₁-C₆ alkoxy-substituted C₁-C₆ alkyl
X is
(a) NH, or
(b) O.

A further embodiment of the present invention is thus to administer to a subject suffering from sleep disturbance due to silent gastro-esophageal reflux a therapeutically effective amount of certain certain imidazo[1,2-a]pyridines derivatives of Formula I wherein R¹ is CH₃ or CH₂OH; R², R³ and R⁴ independently are CH₃ or CH₂CH₃; R⁵ is H, Br, Cl, or F and R⁶, R⁷ are the same or different and chosen from a group consisting of H, C₁-C₆ alkyl, and hydroxylated C₁-C₆ alkyl.

Another embodiment of the present is thus to administer to a subject suffering from sleep disturbance due to silent gastro-esophageal reflux a therapeutically effective amount of any of the following compounds 8-(2-ethyl-6-methylbenzylamino)-3-hydroxymethyl-2-methylimidazo[1,2-a]pyridine-6-carboxamide,
2,3-dimethyl-8-(2,6-dimethylbenzylamino)-N-hydroxyethyl-imidazo[1,2-a]pyridine-6-carboxamide,
2,3-dimethyl-8-(2-ethyl-6-methylbenzylamino)-imidazo[1,2-a]pyridine-6-carboxamide,
8-(2-ethyl-6-methylbenzylamino)-N,2,3-trimethylimidazo[1,2-a]pyridine-6-carboxamide,
2,3-dimethyl-8-(2,6-dimethylbenzylamino)-imidazo[1,2-a]pyridine-6-carboxamide,
2,3-dimethyl-8-(2-ethyl-4-fluoro-6-methylbenzylamino)-imidazo[1,2-a]pyridine-6-carboxamide,
2,3-dimethyl-8-(2,6-dimethyl-4-fluoro-benzylamino)-imidazo[1,2-a]pyridine-6-carboxamide,
2,3-dimethyl-8-(2,6-diethylbenzylamino)-imidazo[1,2-a]pyridine-6-carboxamide,
2,3 dimethyl-8-(2-ethyl-6-methylbenzylamino)-N-hydroxyethyl-imidazo[1,2-a]pyridine-6-carboxamide,
2,3 dimethyl-8-(2-ethyl-6-methylbenzylamino)-N-(2-methoxyethyl)-imidazo[1,2-a]pyridine-6-carboxamide,
or
a pharmaceutically acceptable salt thereof.

In one embodiment of the present invention the therapeutically acceptable salt of a compound of Formula I is the form of a hydrochloride salt.

In another embodiment of the present invention the therapeutically acceptable salt of a compound of Formula I is in the form of a mesylate salt.

The invention relates in a further aspect to the use of P-CABs for the treatment of patients who are suffering from sleep disturbance due to silent gastro-esophageal reflux.

The invention further relates to a method for the treatment of sleep disturbance due to silent gastro-esophageal reflux which consists in administering to a patient who needs such a treatment an effective amount of a P-CAB.

The invention further relates to the use of P-CABs for the production of medicaments for the treatment of sleep disturbance due to silent gastro-esophageal reflux.

The invention further relates to a pharmaceutical preparation for the treatment of sleep disturbance due to silent gastro-esophageal reflux which contains a P-CAB as active compound.

In one embodiment of the present invention the pharmaceutical preparation is intended to give a immediate release profile.

In one embodiment of the present invention the pharmaceutical preparation is intended to give a modified release profile.

The invention further relates to a ready-to-use medicament, comprising a P-CABs as active compound, which contains a reference to the fact that this ready-to-use medicament can be employed for the treatment of sleep disturbance due to silent gastro-esophageal reflux.

Further, it has been found that the reversible proton pump inhibitors that sometimes are referred to as Acid Pump Antagonists (APA), whose original field of use also is the treatment of gastric and intestinal disorders, are suitable for the treatment of sleep disturbance due to silent gastro-esophageal reflux. A further aspect of the present invention is thus the use of reversible proton pump inhibitors in the treatment of sleep disturbance due to silent gastro-esophageal reflux. Reversible proton pump inhibitors are designated as those substances which inhibit gastric acid secretion by blockade of the proton pump, but which, in contrast to the PPls, do not bind covalently to the H'/K+-ATPase, the enzyme responsible for gastric acid secretion. The term "reversible proton pump inhibitor" according to the present invention comprises not only the active compounds as such, but also their pharmacologically acceptable salts and solvates (in particular hydrates) etc.

Reversible proton pump inhibitors are described and claimed, for example, in the following patent applications and patents: EP 33094, EP 204285, EP 228006, EP 233760, EP 259174, EP 266890, EP 270091, EP 307078, EP 308917, EP 330485, US 4728658, US 5362743, WO 9212969, WO 9414795, WO 9418199, WO 9510518, WO 9603405, WO 9605177, WO 9703074, WO 9703076, WO 9642707, WO 9843968, WO 9854188, WO 9909029, WO 9950237, WO 9951584, WO 0001696, WO 0017200, WO 0026217, WO 0029403, WO 0063211, WO 0077003, WO 0158901, WO 0172754, WO 0172755, WO 0172756, WO 0172757, and WO 02034749.

Examples of reversible proton pump inhibitors which can be mentioned on the basis of their (proposed) INNs or their code name are the compounds: AG-2000 (EP 233760), AU-461 (WO 9909029), BY112 (WO 9842707), Soraprazan (BY359) (WO 0017200), CP-113411 (US 5362743), DBM-819 (WO0001696), KR-60436 (WO 9909029), Pumaprazole (WO 9418199), SKF-96067 (EP 259174), SKF-96356 (EP 307078), SKF-97574 (EP 330485), T-330 (EP 270091), T-776 (EP 270091), WY-27198 (US 4728658), YH-1885 (WO 9605177), YJA-20379-8 (WO 9703074), and YM-19020 (EP 266890).

Of these, the compounds AU-461, Soraprazan (BY359), DBM-819, KR-60436, T-330, YH-1885, and YJA-20379-8 are particularly worthy of mention.

A particularly interesting group of reversible proton pump inhibitors is described and claimed in interna-tional patent applications WO 9842707, WO 9854188, WO 0017200, WO 0026217, WO 0063211, WO 0172754, WO 0172755, WO 0172756, WO 0172757 and WO 02034749.

The invention relates in a further aspect to the use of reversible proton pump inhibitors for the treatment of patients who are suffering from sleep disturbance due to silent gastro-esophageal reflux.

The invention further relates to a method for the treatment of sleep disturbance due to silent gastro-esophageal reflux which consists in administering to a patient who needs such a treatment an effective amount of a reversible proton pump inhibitor.

The invention further relates to the use of reversible proton pump inhibitors for the production of medicaments for the treatment of sleep disturbance due to silent gastro-esophageal reflux.

The invention further relates to a pharmaceutical preparation for the treatment of sleep disturbance due to silent gastro-esophageal reflux which contains a reversible proton pump inhibitor as active compound.

In one embodiment of the present invention the pharmaceutical preparation is intended to give a immediate release profile.

In one embodiment of the present invention the pharmaceutical preparation is intended to give a modified release profile.

The invention further relates to a ready-to-use medicament, comprising a reversible proton pump in-hibitor as active compound, which contains a reference to the fact that this ready-to-use medicament can be employed for the treatment of sleep disturbance due to silent gastro-esophageal reflux.

Another aspect of the present is thus to administer to a subject suffering from sleep disturbance due to silent gastro-esophageal reflux a therapeutically effective amount of soraprazan.

*The dosage form or forms, methods for preparing the pharmaceutical formulation and methods of* *administering the active ingredient and*/*or pharmaceutical formulation, including dosage levels and frequency,*

### IMMEDIATE-RELEASE FORMULATIONS

According to a one embodiment of the invention, there is provided an immediate-release pharmaceutical formulation comprising:
(a) an active ingredient (chosen from any of the compounds listed above), or a pharmaceutically-acceptable salt of any of these compounds; and
(b) a pharmaceutically-acceptable diluent or carrier,
which formulations are referred to hereinafter as "the immediate-release formulations of the invention".

The term "immediate release" pharmaceutical formulation will be well understood by the skilled person to include any formulation in which the onset and/or rate of release, and/or absorption, of drug, is neither appreciably, nor intentionally, retarded by galenic manipulations. In the present case, immediate release may be provided for by way of an appropriate pharmaceutically-acceptable diluent or carrier, which diluent or carrier does not prolong, to an appreciable extent, the onset and/or rate of drug release/absorption. Thus, the term will be understood by those skilled in the art to exclude formulations which are adapted to provide for "modified" or "controlled" release, including a "sustained", "prolonged", "extended" or "delayed" release of drug.

In this context, the term "release" may be understood to include provision (or presentation) of drug from the formulation to the gastrointestinal tract, to body tissues and/or into systemic circulation.

Thus, immediate-release formulations of the invention may release at least 70% (e.g. 80%) of active ingredient within 4 hours, such as within 3 hours, preferably 2 hours, more preferably within 1.5 hours, and especially within an hour (such as within 30 minutes), of administration, whether this be oral or parenteral.

The immediate-release formulations of the invention may be formulated in accordance with a variety of techniques known to those skilled in the art, for example as described by M. E. Aulton in "Pharmaceutics: The Science of Dosage Form Design" (1988) (Churchill Livingstone), the relevant disclosures in which document are hereby incorporated by reference.

Immediate-release formulations of the invention may be, or may be adapted in accordance with standard techniques to be, suitable for peroral administration, for example in the form of immediate release tablets or capsules, or as liquid dosage forms, comprising active ingredient, both of which formulation types are well known to the skilled person and may be prepared in accordance with techniques known in the art.

Immediate-release formulations of the invention in the form of for example immediate release tablets may further comprise one or more additional excipients known to those skilled in the art for use in such immediate-release formulations, to improve the physical and/or chemical properties of the final composition, and/or to facilitate the process of manufacture. Combinations of excipients may be employed.

Alternatively, immediate-release formulations of the invention may be, or may preferably be adapted in accordance with standard techniques to be, suitable for parenteral administration. The term "parenteral" will be understood by those skilled in the art to include any mode of administration that does not comprise peroral administration to the gastrointestinal tract. The term may thus be understood to include administration subcutaneously, intravenously, intraarterially, transdermally, intranasally, intrabuccally, intracutaneously, intramuscularly, intralipomateously, intraperitoneally, rectally, sublingually, topically, by inhalation, or by any other parenteral route.

Suitable immediate-release formulations of the invention that are to be administered parenterally include those in which an active ingredient (chosen from any of the compounds listed above), or pharmaceutically-acceptable salts thereof, are presented together with an aqueous carrier, such as water.

Immediate-release formulations of the invention comprising aqueous carriers may further comprise one or more additional excipients known to those skilled in the art for use in aqueous parenteral immediate-release formulations, such as antimicrobial preservatives; tonicity modifiers (such as sodium chloride, mannitol, glucose and the like); pH adjusting agents (such as common inorganic acids and bases, including hydrochloric acid, sodium hydroxide and the like); pH controlling agents (i.e. buffers, such as tartaric acid, acetic acid, citric acid and the like); surfactants (e.g. SolutolTM); cosolvents, which may serve to further solubilise active ingredient (such as ethanol, polyethylene glycols, hydroxypropyl-b-cyclodextrin and the like); and/or antioxidants.

The amount of further excipients that may be employed in the peroral and parenteral immediate-release formulations of the invention depends upon many factors, including the nature and amount of active ingredient that is included, and the amount of diluent/carrier (aqueous solvent or otherwise) that is included, but may be in line with those amounts that are typically employed in immediate release pharmaceutical formulations that are known in the art, and/or may be determined routinely by the skilled person.

Immediate-release formulations of the invention that may be, for example, suitable for parenteral administration, may be provided in the form of suspensions of active ingredient in association with an aqueous solvent or, more preferably, and especially when the formulation is to be administered parenterally (particularly intravenously), aqueous solutions (i.e. solutions of active compound including water as a solvent). In this context, the term "aqueous solution" may be understood to include immediate-release formulations in which at least 99% of active ingredient is in solution at above 5°C and atmospheric pressure, and the term "suspension" should be construed accordingly (i.e. more than 1% of active ingredient is not in solution under such conditions).

Thus, pharmaceutically-acceptable salts of the active ingredient which may be particularly mentioned include those of inorganic and organic acids, which acids may form watersoluble salts when added to an active ingredient (chosen from any of the compounds listed above),. It will be appreciated by the skilled person that water solubility of salts that may be formed between acids and an active ingredient (chosen from any of the compounds listed above), may depend upon the physical and chemical properties of the acid, including inter alia the water solubility (of the acid itself), lipophilicity of the counter-ion and the acid's dissociation constant.

Immediate-release formulations of the invention that are in the form of immediate release tablets may be prepared by bringing active ingredient into association with diluent/carrier using standard techniques, and using standard equipment, known to the skilled person, including wet or dry granulation, direct compression/compaction, drying, milling, mixing, tabletting and coating, as well as combinations of these processes, for example as described hereinafter.

For parenteral immediate-release formulations of the invention, it is preferred that the immediate-release formulations of the invention comprising active ingredient in the form of free base are prepared by adding free base to an appropriate diluent/carrier (e.g. solvent system comprising water). It is preferred that such immediate-release formulations of the invention comprising active ingredient in the form of an acid addition salt are prepared by addition of acid (either directly or in association with a diluent/carrier, such as in the form of a solution (such as an aqueous solution) of appropriate acid) to base, which base may, for example be provided in association with a diluent/carrier, such as in the form of a solution (such as an aqueous solution), followed by addition of further diluent/carrier/solvent, if and as appropriate.

The skilled person will appreciate that appropriate additional excipients may be added at a suitable stage in the preparation of the formulation of the invention (i.e. before, during or after the process of bringing active ingredient into association with diluent/carrier). For example, for parenteral immediate-release formulations of the invention in the form of aqueous solutions, the pH of the mixture of an active ingredient or salt thereof and diluent/carrier may be controlled by addition of an appropriate buffer and/or adjusted by way of a pH adjusting agent, for example as described hereinafter.

Immediate-release formulations of the invention (particularly those suitable for eventual parenteral administration) in the form of a suspension, or particularly a solution, such as an aqueous solution, may be provided in "ready-to-use" form, by which we include in a form that is suitable for administration directly to a patient, with the aid of a suitable dosing means, without further preparative or formulative work being necessary.

However, such immediate-release formulations (i.e. those in the form of a suspension or solution, particularly an aqueous solution) may also be provided in the form of a "concentrate" of active ingredient and diluent/carrier. Immediate-release formulations in this form, hereinafter referred to as "concentrated immediate-release formulations of the invention" or "concentrates", may thus be employed in the preparation of a corresponding formulation of the invention suitable for e.g. parenteral administration by addition of further diluent/carrier (and, if appropriate, further excipients), prior to administration to a patient. For example, aqueous concentrates, particularly for use in parenteral immediate-release formulations, may be prepared ready for reconstitution and/or dilution (e.g. by addition of water, physiological saline, glucose solution or any other suitable solution) prior to administration.

Concentrated immediate-release formulations of the invention may be prepared directly by bringing diluent or carrier (and, if appropriate, additional excipients) into association with the active ingredient as described hereinbefore. Concentrates may also be prepared by preparation of a formulation of the invention in the form of e.g. an aqueous solution, as hereinbefore described, which may include additional excipients, followed by removal of pharmaceutically-acceptable diluent or carrier (e.g. solvent, such as aqueous solvent). Solvents may be removed by way of a variety of techniques known to those skilled in the art, for example evaporation (under reduced pressure or otherwise). Thus, immediate-release formulations of the invention (e.g. parenteral formulations) in ready-to-use form may also be provided by addition of diluent or carrier (and, if appropriate, further excipients) to a concentrated formulation of the invention.

The amount of diluent/carrier in an oral (e.g. immediate release tablet) formulation of the invention depends upon many factors, such as the nature and amount of the active ingredient that is employed and the nature, and amounts, of any other constituents (e.g. further excipients) that are present in the formulation, but is typically up to 40% (w/w), preferably up to 30%, more preferably up to 20%, and particularly up to 10% (w/w) of the final composition. The amount of additional excipients in such an oral formulation of the invention also depends upon factors, such as the nature and amount of the active ingredient that is employed, as well as the nature, and amounts, of any other constituents (e.g. diluents/carriers and/or other further excipients) that are present in the formulation, but, for lubricants and glidants is typically up to 5% (w/w), and for binders and disintegrants is typically up to 10% (w/w) of the final composition.

The amount of diluent/carrier in a "ready-to-use" parenteral formulation of the invention as defined above depends upon many factors, such as the nature and amount of the active ingredient that is employed and the nature, and amounts, of any other constituents (e.g. further excipients) that are present in the formulation, but is typically at least 50% (w/w) of the final composition. In concentrated immediate-release formulations of the invention, the amount of diluent/carrier is typically at least 10% (w/w) of the concentrate. (It is to be noted, however, that although these lower limits for diluent/carrier content are typical, they may not always be applicable, for example in cases where the solubility of active ingredient in the relevant diluent/carrier is particularly high.)

Compositions including active ingredient may also be provided in solid form suitable for use in the preparation of a immediate-release formulation of the invention (e.g. a solution, such as an aqueous solution, for example for parenteral adminstration) ex tempore.

Such compositions may be in the form of a solid comprising active ingredient, optionally in the presence of one or more further excipients as hereinbefore defined and, optionally, up to 10% (w/w) of diluent and/or carrier as hereinbefore defined, which compositions are hereinafter referred to as "the solid compositions of the invention".

Solid compositions of the invention may be made by removal of diluent/carrier (e.g. solvent) from a immediate-release formulation of the invention, or a concentrated f immediate-release ormulation of the invention, which may for example be in the form of a solution, such as an aqueous solution.

There is thus provided a process for the formation of a solid composition suitable for use in the preparation of a immediate-release formulation of the invention (e.g. a solution, such as an aqueous solution) ex tempore, which process comprises removal of diluent/carrier (e.g. solvent) from a immediate-release formulation of the invention, or a concentrated immediate-release formulation of the invention.

Solvent may be removed by way of a variety of techniques known to those skilled in the art, for example evaporation (under reduced pressure or otherwise), freeze-drying, or any solvent removal (drying) process that removes solvent (such as water) while maintaining the integrity of the active ingredient. Freeze-drying is preferred.

Thus according to a further aspect of the invention there is provided a freeze-dried (lyophilised) solid composition of the invention.

In the preparation of solid compositions of the invention, the skilled person will appreciate that appropriate additional excipients may be added at a suitable stage prior to removal of diluent/carrier. For example, in the case of aqueous solutions, pH may be controlled and/or adjusted as hereinbefore described. Furthermore, an appropriate additional excipient may be added with a view to aiding the formation of a solid composition of the invention during the process of diluent/carrier removal.

Solid compositions of an active ingredient, or salts thereof, thus include those in which the solvent (e.g. water) content, other than solvents of crystallisation is no more than 10%, such as less than 2% unbound solvent, such as water.

Immediate-release formulations of the invention may be sterilised, for example by sterile filtration or autoclavation, and/or filled into primary packages, such as vials, cartridges and pre-filled syringes. Such processing steps may also take place prior to drying to form a solid composition of the invention.

Before administration, the dried solid composition may be reconstituted and/or diluted in, for instance, water, physiological saline, glucose solution or any other suitable solution.

Typical daily doses of the active ingredient, or pharmaceutically-acceptable salts thereof, are in the range 5 to 1000 mg, irrespective of the number of individual doses that are administered during the course of that day. Preferred daily doses are in the range 10 to 100 mg and more preferred daily doses are in the range of 20 to 80 mg, e.g. 50 mg.

### MODIFIED RELEASE

According to the invention there is provided a modified release pharmaceutical formulation comprising, an active ingredient, or a pharmaceutically-acceptable salt of an active ingredient, which formulations are referred to hereinafter as "the modified release formulations of the invention".

The term "modified release" pharmaceutical formulation will be well understood by the skilled person to include any modified release formulation in which the onset and/or rate of release of drug is altered by galenic manipulations, and thus includes the definition provided in the United States Pharmacopeia (USP XXII) at pages xliii and xliv of the preface/preamble part, the relevant disclosure in which document is hereby incorporated by reference.

In the present case, modified release may be provided for by way of an appropriate pharmaceutically-acceptable carrier, and/or other means, which carrier or means (as appropriate) gives rise to an alteration of the onset and/or rate of release of active ingredient. Thus, the term will be understood by those skilled in the art to include modified release formulations which are adapted (for example as described herein) to provide for a "sustained", a "prolonged" or an "extended" release of drug (in which drug is released at a sufficiently retarded rate to produce a therapeutic response over a required period of time, optionally including provision for an initial amount of drug being made available within a predetermined time following administration to cause an initial desired therapeutic response); modified release formulations which provide for a "delayed" release of drug (in which the release of drug is delayed until a specific region of the gastrointestinal tract is reached, following which drug release may be either pulsatile or further modified as indicated above); as well as so-called "repeat action" formulations (in which one dose of drug is released either immediately or some time after administration and further doses are released at a later time).

We prefer that the modified release formulations of the invention provide for a delayed release or, more preferably, a sustained (i.e. prolonged or extended) release of drug over a period of time. More preferred modified release formulations of the invention may be adapted (for example as described herein) to provide a sufficient dose of drug over the dosing interval (irrespective of the number of doses per unit time) to produce a desired therapeutic effect. Release may be uniform and/or constant over an extended period of time, or otherwise.

Modified release formulations of the invention may, for example, be in the form of the following, all of which are well known to those skilled in the art:
(a) Coated pellets, tablets or capsules, which may be designed to release at least some of the drug when the modified release formulation in question reaches a particular region of the gastrointestinal tract. Such tablets may, for example be provided with some form of gastro-resistant coating, such as an enteric coating layer, providing for release of at least part of the drug present in the modified release formulation in a specific part of the gastrointestinal tract, such as the intestinal regions.
(b) Multiple unit or multiparticulate systems, which may be in the form of microparticles, microspheres or pellets comprising drug (which multiple units/multiparticulates may provide for gradual emptying of the modified release formulation containing drug from the stomach into the duodenum and further through the small and large intestine while releasing drug at a pre-determined rate).
(c) Modified release formulations comprising dispersions or solid solutions of active compound in a matrix, which may be in the form of a wax, gum or fat, or, particularly, in the form of a polymer, in which drug release takes place by way of gradual surface erosion of the tablet and/or diffusion.
(d) Systems which comprise a bioadhesive layer, which layer may provide for prolonged retention of modified release formulation of the invention in a particular region of the gastrointestinal tract (e.g. the stomach). This includes floating or sinking systems (i.e. low and high density systems, respectively), as well as so-called "volume-enlarging" systems.
(e) So-called "pendent" devices, in which drug is attached to an ion exchange resin, which provides for gradual release of drug by way of influence of other ions present in the gastrointestinal tract, for example, the acid environment of the stomach.
(f) Devices in which release rate of drug is controlled by way of its chemical potential (e.g. the Osmotic Pump).
(g) Systems in which drug is released by diffusion through membranes, including multilayer systems.
(h) Devices that act in accordance with an external signal, to release a small amount of drug.
(i) Active, self-programmed systems, which may contain a sensing element, which element responds to a particular biological environment to modulate drug delivery.
(j) Silastic controlled release depots, which release drug as a function of diffusion of water and/or gastrointestinal fluids into the device via an entry/exit port, resulting in dissolution and subsequent release of drug.
(k) Combinations of two or more of the above principles.

The above principles are discussed at length in numerous prior art references including Pharmaceutisch Weekblad Scientific Edition, 6, 57 (1984); Medical Applications of Controlled Release, Vol II, eds. Langer and Wise (1984) Bocaraton, Florida, at pages 1 to 34; Industrial Aspects of Pharmaceuticals, ed. Sandel, Swedish Pharmaceutical Press (1993) at pages 93 to 104; and pages 191 to 211 of "Pharmaceutics: The Science of Dosage Form Design", ed. M. E. Aulton (1988) (Churchill Livingstone); as well as the references cited in the above-mentioned documents, the disclosures in all of which documents are hereby incorporated by reference.

Suitable modified release formulations may thus be prepared by the skilled person in accordance with standard techniques in pharmacy, as described herein or in the above-mentioned documents, and/or which are well known.

One object of the present invention is a modified release formulations of the invention wherein the active ingredient is embedded in a polymer matrix.

In this respect, we prefer that the modified release formulations of the invention are provided for oral administration in the form of a so-called "swelling" modified-release system, or a "gelling matrix" modified-release system, in which active ingredient is provided together with a polymer that swells in an aqueous medium (i.e. a "hydrophilic gelling component"). The term "aqueous medium" is to be understood in this context to include water, and liquids which are, or which approximate to, those present in the gastrointestinal tract of a mammal. Such polymer systems typically comprise hydrophilic macromolecular structures, which in a dry form may be in a glassy, or at least partially crystalline, state, and which swell when contacted with aqueous media. Modified release of drug is thus effected by one or more of the following processes: transport of solvent into the polymer matrix, swelling of the polymer, diffusion of drug through the swollen polymer and/or erosion of the polymer, one or more of which may serve to release drug slowly from the polymer matrix into an aqueous medium.

Thus, suitable polymeric materials (i.e. carriers), which may be used as the hydrophilic gelling component of a gelling matrix modified-release formulation include those with a molecular weight of above 5000 g/mol, and which either:
(a) are at least sparingly soluble in; or
(b) swell when placed in contact with,
aqueous media (as defined hereinbefore), so enabling release of drug from the carrier.

The choice of polymer will be determined by the nature of the active ingredient/drug that is employed in the modified release formulation of the invention as well as the desired rate of release. In particular, it will be appreciated by the skilled person. In this respect, and as stated above, it may be desirable to provide modified release formulations of the invention in the form of gelling matrix systems in which the polymer carrier is provided by way of a blend of two or more polymers of, for example, different molecular weights in order to produce a particular required or desired release profile.

When in the form of gelling matrix systems, we have also found that rate of release of drug from modified release formulations of the invention may be further controlled by way of controlling the drug:polymer ratio within, and the surface area:volume ratio of, individual modified release formulations (e.g. tablets) comprising drug and polymer carrier system.

Modified release formulations of the invention, whether in the form of a gelling matrix system or otherwise, may contain one or more further excipients (in addition to the polymer carrier system) to further modify drug release, to improve the physical and/or chemical properties of the final composition, and/or to facilitate the process of manufacture. Such excipients are conventional in the formulation of modified release compositions.

Modified release formulations of the invention may contain one or more lubricants.

Modified release formulations of the invention may contain a glidant.

Other further excipients may include colourants, flavourings, tonicity-modifying agents, coating agents, preservatives, etc.

Combinations of the above-stated further excipients may be employed.

It will be appreciated by the skilled person that some of the above mentioned further excipients, which may be present in the final composition of the invention, may have more than one of the above-stated functions. Moreover, further excipients mentioned above may also function as part of a hydrophilic gelling component in a gelling matrix system.

The total amount of further excipients (not including, in the case of gelling matrix systems, the principal polymer carrier) that may be present in the modified release formulation of the invention will depend upon the nature of the modified release formulation, as well as the nature, and amounts of, the other constituents of that modified release formulation, and may be an amount of up to 85%, for example between 0.1 to 75%, such as 0.2 to 65%, preferably 0.3 to 55%, more preferably 0.5 to 45% and especially 1 to 40%, such as 2 to 35% w/w. In any event, the choice, and amount, of excipient(s) may be determined routinely (i.e. without recourse to inventive input) by the skilled person.

In gelling matrix systems, the amount of polymer in the system should be enough to ensure that a sufficient dose of drug is provided over the dosing interval to produce the desired therapeutic effect. Thus, we prefer that at least 60% (such as 80%) of the initial drug content of the modified release formulation is released to a patient, and/or under the test conditions described hereinafter, over a period of 2 hours or longer, preferably a period of 4 hours or longer, more preferably a period of 6 hours or longer and particularly over a period of between 8 and 24 hours. Suitable amounts of polymer that may be included, which will depend upon inter alia the active ingredient that is employed in the modified release formulation, any excipients that may be present and the nature of the polymer that is employed, are in the range 5 to 99.5%, for example 10 to 95%, particularly 15 to 80%, preferably 20 to 75%, more preferably 30 to 70% and especially 35 to 65% w/w. In any event, the choice, and amount, of polymer may be determined routinely by the skilled person.

When modified release formulations of the invention are provided in the form of gelling matrix systems, an active ingredient that may be mentioned include the free base forms of an active ingredient, as well as salts in which the solubility of that salt in aqueous media (as defined above) is substantially independent of the pH of that medium, particularly pHs in the physiological range typically found in the gastrointestinal tract.

Suitable amounts of active ingredient in the modified release formulations of the invention, whether in the form of gelling matrix systems or otherwise, depend upon many factors, such as the nature of that ingredient (free base/salt etc), the dose that is required, and the nature, and amounts, of other constituents of the modified release formulation. However, they may be in the range 0.5 to 80%, for example 1 to 75%, such as 3 to 70%, preferably 5 to 65%, more preferably 10 to 60% and especially 15 to 55% w/w. In any event, the amount of active ingredient to be included may be determined routinely by the skilled person.

Typical daily doses of an active ingredient, or pharmaceutically-acceptable salts of any of these compounds, are in the range 10 to 2000 mg, e.g. 25, such as 30, to 1200 mg of free base (i.e., in the case of a salt, excluding any weight resulting from the presence of a counter ion), irrespective of the number of modified release formulations (e.g. tablets) that are administered during the course of that day. Preferred daily doses are in the range 50 to 1000 mg, such as 100 to 500 mg. Typical doses in individual modified release formulations of the invention (e.g. tablets) are thus in the range 15 to 500 mg, for example 40 to 400 mg.

Modified release formulations of the invention such as those described hereinbefore may be made in accordance with well known techniques such as those described in the references mentioned hereinbefore. Modified release formulations of the invention that are in the form of gelling matrix systems may be prepared by standard techniques, and using standard equipment, known to the skilled person, including wet or dry granulation, direct compression/compaction, drying, milling, mixing, tabletting and coating, as well as combinations of these processes, for example as described hereinafter.

Although modified release formulations of the invention are preferably adapted to be administered orally, their use is not limited to that mode of administration. Parenteral modified release formulations of the invention, which may include systems that are well known to those skilled in the art, such as those based upon poloxamers, biodegradable microspheres, liposomes, suspensions in oils and/or emulsions, may be prepared in accordance with standard techniques, for example as described by Leung et al in "Controlled Drug Delivery: Fundamentals and Applications" (Drugs and the Pharmaceutical Sciences; vol. 29), 2nd edition, eds. Robinson and Lee, Dekker (1987) at Chapter 10, page 433, the disclosure in which document is hereby incorporated by reference.

The modified release formulations of the invention may be dosed once or more times at bedtime (e.g. up to six times, but preferably no more than twice), irrespective of the number of individual units (formulations) that are administered as part of one "dose".

For the avoidance of doubt, by "treatment" we include the therapeutic treatment, as well as the prophylaxis, of a condition.

Modified release formulations of the invention have the advantage that they may provide a modified release of active ingredient or a pharmaceutically-acceptable salt of any of these compounds, in order to obtain a more even and/or prolonged effect. Certain modified release formulations of the invention may achieve this release in an essentially pH-independent manner.

Modified release formulations of the invention may also have the advantage that they may be prepared using established pharmaceutical processing methods and employ materials that are approved for use in foods or pharmaceuticals or of like regulatory status.

For the avoidance of doubt, by "treatment" we include the therapeutic treatment, as well as the prophylaxis, of a condition.

One aspect of the present invention is to administer a pharmaceutically active amount of the active ingredient at bedtime.

Advantages with the present invention includes, but are not limited to, limiting the use of hypnotics to treat sleep disturbance, limiting the amount of fluid excreted by the stomach, reducing the intervariability between patients, more effective acid secretion inhibition than therapeutic amounts of other drugs with this effect (proton pump inhibitors).

Typical daily doses of the active ingredient, or pharmaceutically-acceptable salts thereof, are in the range 5 to 1000 mg, irrespective of the number of individual doses that are administered during the course of that day. Preferred daily doses are in the range 10 to 100 mg and more preferred daily doses are in the range of 20 to 80 mg, e.g. 50 mg.

### Examples

The following example is intended just as an Example and should not be seen as limiting the present invention.

### Example 1.

Subjects meeting the entry criteria will undergo a history and physical exam. All subjects will undergo a standard drug screening test. Subjects will complete the Pittsburgh Sleep Quality Index, Functional Outcomes of Sleep Questionnaire, the Beck Depression Inventory, and the SF-36 for assessment of quality of life. All subjects will complete a daily sleep log for two weeks. At the end of this "run in" interval, an assessment will be made of the nights of disturbed or mornings with unrefreshed sleep. Subsequent to qualification, all subjects will undergo a full polysomnography (PSG) to include esophageal pH monitoring. All subjects will complete a questionnaire prior to bedtime and upon awakening in the morning. They are designed to assess acitivites of the current day and mental state prior to the sleep study, as well as morning mental state and subjective reports of awakenings and heartburn symptoms experienced during the sleep study.

The subject are then randomized into two groups, one that will get the active compound and one that will get placebo.

### PSG Study:

The PSG study will consist of monitoring the EEG, EOG, EMG, and EKG. Respiration will be assessed via nasal oral sensor. The following parameters will be determined using standard internationally accepted criteria:
- Total sleep time (TST)
- Sleep onset latency (SOL)
- Sleep efficiency (time asleep/time in bed)
- Waking after sleep onset (WASO)
- Arousal reponses (arousal responses will be defined by current AASM practice guidelines criteria).
- Percent time REM sleep
- Percent stage 3 and 4

### Esophageal pH Study:

A standard pH probe with dual sensors will be placed 5 cm above the manometrically determined proximal border of the lower esophageal sphincter (LES). The second pH sensor will be 5 cm proximal to the distal sensor. This will be accomplished at approximately 4:00 in the afternoon prior to each PSG study. The following pH parameters will be assessed:
- Number of reflux events at the distal and proximal pH sensor
- Arousal responses associated with reflux events (arousal responses will be defined as occurring within 5 minutes subsequent to the fall in pH below 4.0)
- Average clearance time/event
- Percentage acid contact time
- Events exceeding 5 minutes duration

### DATA ANALYSIS

Data analysis will consist of comparing the two randomized groups, one on drug and one of placebo. The outcome will be compared in regard to the test discussed above, i.a. Pittsburgh Sleep Quality Index, Functional Outcomes of Sleep Questionnaire, the Beck Depression Inventory, the SF-36 for assessment of quality of life, Phagosonogram (PSG), Quality of Life in Reflux and Dyspepsia (QOLRAD) and esophageal pH.

## Claims

1. Use of a compound of Formula I for the production of medicaments for the treatment of sleep disturbance due to silent gastro-esophageal reflux 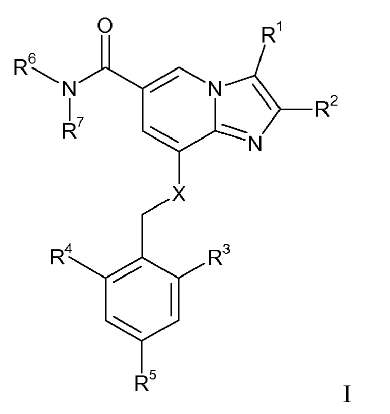 R¹ is
(a) H,
(b) CH₃, or
(c) CH₂OH;
R² is
(a) CH₃
(b) CH₂CH₃;
R³ is
(a) H
(b) C₁-C₆ alkyl,
(c) hydroxylated C₁-C₆ alkyl
(d) halogen;
R⁴ is
(a) H,
(b)C₁-C₆ alkyl,
(c) hydroxylated C₁-C₆ alkyl, or
(d) halogen;
R⁵ is
(a) H, or
(b) halogen;
R⁶, R⁷ are the same or different
(a) H,
(b) C₁-C₆ alkyl;
(c) hydroxylated C₁-C₆ alkyl
(d) C₁-C₆ alkoxy-substituted C₁-C₆ alkyl;
X is
(a) NH, or
(b) O;
or a pharmaceutically acceptable salt thereof.

2. Use according to claim 1, wherein R¹ is CH₃ or CH₂OH; R², R³ and R⁴ independently are CH₃ or CH₂CH₃; R⁵ is H, Br, Cl, or F and R⁶, R⁷ are the same or different and chosen from a group consisting of H, C₁-C₆ alkyl, and hydroxylated C₁-C₆ alkyl; or a pharmaceutically acceptable salt thereof.

3. Use according to anyone of claims 1 - 2, wherein the compound of Formula I is selected from a group consisting of
8-(2-ethyl-6-methylbenzylamino)-3-hydroxymethyl-2-methylimidazo[1,2-a]pyridine-6-carboxamide,
2,3-dimethyl-8-(2,6-dimethylbenzylamino)-N-hydroxyethyl-imidazo[1,2-a]pyridine-6-carboxamide,
2,3-dimethyl-8-(2-ethyl-6-methylbenzylamino)-imidazo[1,2-a]pyridine-6-carboxamide,
8-(2-ethyl-6-methylbenzylamino)-N,2,3-trimethylimidazo[1,2-a]pyridine-6-carboxamide,
2,3-dimethyl-8-(2,6-dimethylbenzylamino)-imidazo[1,2-a]pyridine-6-carboxamide,
2,3-dimethyl-8-(2-ethyl-4-fluoro-6-methylbenzylamino)-imidazo[1,2-a]pyridine-6-carboxamide,
2,3-dimethyl-8-(2,6-dimethyl-4-fluoro-benzylamino)-imidazo[1,2-a]pyridine-6-carboxamide,
2,3-dimethyl-8-(2,6-diethylbenzylamino)-imidazo[1,2-a]pyridine-6-carboxamide,
2,3-dimethyl-8-(2-ethyl-6-methylbenzylamino)-N-hydroxyethyl-imidazo[1,2-a]pyridine-6-carboxamide, and
2,3-dimethyl-8-(2-ethyl-6-methylbenzylamino)-N-(2-methoxyethyl)-imidazo[1,2-a]pyridine-6-carboxamide,
or a pharmacologically acceptable salt thereof.

4. Use according to anyone of claims 1-3, wherein the compound of Formula I is in the form of hydrochloride or mesylate salt.

5. Use of a reversible proton pump inhibitor for the production of medicaments for the treatment of sleep disturbance due to silent gastro-esophageal reflux, wherein the reversible proton pump inhibitor is soraprazan.

6. Pharmaceutical preparation for use in the treatment of sleep disturbance due to silent gastro-esophageal reflux, containing a compound of Formula I as active compound together with a pharmaceutically acceptabler diluent or carrier 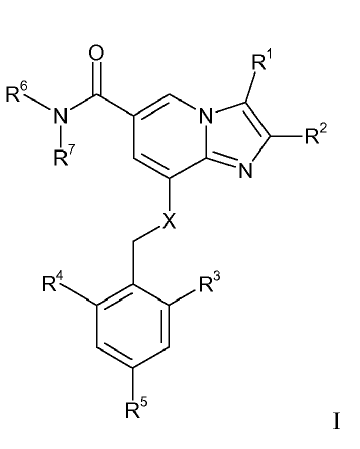 R¹ is
(a) H,
(b) CH₃, or
(c) CH₂OH;
R² is
(a) CH₃
(b) CH₂CH₃;
R³ is
(a) H
(b) C₁-C₆ alkyl,
(c) hydroxylated C₁-C₆ alkyl
(d) halogen;
R⁴ is
(a) H,
(b) C₁-C₆ alkyl,
(c) hydroxylated C₁-C₆ alkyl, or
(d) halogen;
R⁵ is
(a) H, or
(b) halogen;
R⁶, R⁷ are the same or different
(a) H,
(b) C₁-C₆ alkyl;
(c) hydroxylated C₁-C₆ alkyl
(d) C₁-C₆ alkoxy-substituted C₁-C₆ alkyl;
X is
(a) NH, or
(b) O;
or a pharmaceutically acceptable salt thereof.

7. Pharmaceutical preparation according to claim 6, wherein R¹ is CH₃ or CH₂OH; R², R³ and R⁴ independently are CH₃ or CH₂CH₃; R⁵ is H, Br, Cl, or F and R⁶, R⁷ are the same or different and chosen from a group consisting of H, C₁-C₆ alkyl, and hydroxylated C₁-C₆ alkyl; or a pharmaceutically acceptable salt thereof.

8. Pharmaceutical preparation according to anyone of claims 6-7, wherein the compound of Formula I is selected from a group consisting of
8-(2-ethyl-6-methylbenzylamino)-3-hydroxymethyl-2-methylimidazo[1,2-a]pyridine-6-carboxamide,
2,3-dimethyl-8-(2,6-dimethylbenzylamino)-N-hydroxyethyl-imidazo[1,2-a]pyridine-6-carboxamide,
2,3-dimethyl-8-(2-ethyl-6-methylbenzylamino)-imidazo[1,2-a]pyridine-6-carboxamide,
8-(2-ethyl-6-methylbenzylamino)-N,2,3-trimethylimidazo[1,2-a]pyridine-6-carboxamide,
2,3-dimethyl-8-(2,6-dimethylbenzylamino)-imidazo[1,2-a]pyridine-6-carboxamide,
2,3-dimethyl-8-(2-ethyl-4-fluoro-6-methylbenzylamino)-imidazo[1,2-a]pyridine-6-carboxamide,
2,3-dimethyl-8-(2,6-dimethyl-4-fluoro-benzylamino)-imidazo [1,2-a]pyridine-6-carboxamide,
2,3-dimethyl-8-(2,6-diethylbenzylamino)-imidazo [1,2-a]pyridine-6-carboxamide,
2,3-dimethyl-8-(2-ethyl-6-methylbenzylamino)-N-hydroxyethyl-imidazo[1,2-a]pyridine-6-carboxamide, and
2,3-dimethyl-8-(2-ethyl-6-methylbenzylamino)-N-(2-methoxyethyl)-imidazo[1,2-a]pyridine-6-carboxamide,
or a pharmacologically acceptable salt thereof.

9. Pharmaceutical preparation according to anyone of claims 6-8, wherein the compound of Formula I is in the form of hydrochloride or mesylate salt.

10. Pharmaceutical preparation according to anyone of claims 6 - 9, suitable for immediate-release.

11. Pharmaceutical preparation according to claim 6 - 9, suitable for modified release.

12. Pharmaceutical preparation for use in the treatment of sleep disturbance due to silent gastro-esophageal reflux, containing a reversible proton pump inhibitor as active compound together with a pharmaceutically acceptabler diluent or carrier, wherein the reversible proton pump inhibitor is soraprazan.
